# EUROPÄISCHE PATENTSCHRIFT

(11) **EP 3 041 439 B1**
(45) Veröffentlichungstag und Bekanntmachung des Hinweises auf die Patenterteilung: **09.05.2018**
(21) Anmeldenummer: 14777276.8
(22) Anmeldetag: 03.09.2014
(51) Int. Cl.: A61F 2/966

(54) **EINFÜHR- UND ABLÖSESYSTEM FÜR IMPLANTATE**
INSERTION AND RELEASE SYSTEM FOR IMPLANTS
SYSTÈME D'INSERTION ET DE RETRAIT D'IMPLANTS

(30) Priorität: 03.09.2013 DE 102013014523
(43) Veröffentlichungstag der Anmeldung: 13.07.2016
(73) Patentinhaber: Phenox GmbH, 44801 Bochum (DE)
(72) Erfinder: MONSTADT, Hermann, 44797 Bochum (DE); HANNES, Ralf, 44137 Dortmund (DE); STATECZNY, Diana, 44791 Bochum (DE); ROLLA, Stefan, 44869 Bochum (DE); SALIN, Manuel, 44801 Bochum (DE)
(74) Vertreter: Schneiders, Josef
(86) Internationale Anmeldenummer: PCT/EP2014/068691
(87) Internationale Veröffentlichungsnummer: WO 2015/032798

(56) Entgegenhaltungen:
- WO-A1-2006/105500
- US-A1- 2006 259 120
- US-A1- 2011 251 666
- US-A1- 2012 303 111

## Beschreibung

Die Erfindung betrifft eine Vorrichtung zur Einbringung eines Implantats für Blutgefäße in den menschlichen Körper, umfassend ein Implantat, einen Einführdraht und eine schlauchartige Umhüllung, wobei das Implantat in der Weise verformbar ist, dass es in einem Mikrokatheter eine Form mit vermindertem Durchmesser annimmt und am Ort der Implantation nach Wegfall des äußeren Zwangs durch den Mikrokatheter unter Anpassung an den Blutgefäßdurchmesser expandiert, das Implantat am proximalen Ende über Verbindungselemente zu einem Halteelement verfügt, über welches das Implantat an den Einführdraht gekoppelt ist, und das Halteelement peripher Ausnehmungen aufweist, in die die Verbindungselemente eingepasst sind, wobei die schlauchartige Umhüllung formschlüssig über das Halteelement mit den eingepassten Verbindungselementen gezogen ist, so dass die Verbindungselemente in den Ausnehmungen des Halteelements fixiert werden und durch Zurückziehen der schlauchartigen Umhüllung in proximaler Richtung eine Ablösung des Implantats erfolgt.

Arteriovenöse Fehlbildungen können in einem Patienten zu erheblichen Beeinträchtigungen und Gefährdungen bis hin zum Tode führen. Dies gilt insbesondere für arteriovenöse Fisteln und Aneurysmen, besonders dann, wenn sie im zerebralen Bereich auftreten. In der Regel versucht man, solche Fehlbildungen durch Implantate zu verschließen. Derartige Implantate werden in der Regel auf endovaskulärem Weg mit Hilfe von Kathetern gesetzt.

Insbesondere bei Aneurysmen hat sich die Implantierung von Platinspiralen bewährt, die das Aneurysma mehr oder weniger vollständig ausfüllen, den Bluteinstrom weitgehend blockieren und dazu führen, dass sich ein lokaler Thrombus ausbildet, der das Aneurysma ausfüllt und letztlich verschließt. Diese Behandlungsmethode ist allerdings nur für Aneurysmen geeignet, die über einen relativ engen Zugang zum Gefäßsystem verfügen, sogenannten Beerenaneurysmen. Bei Aussackungen von Blutgefäßen, die über einen weiten Zugang zum Gefäß verfügen, drohen die implantierten Spiralen wieder ausgeschwemmt zu werden und Schäden in anderen Bereichen des Gefäßsystems herbeizuführen.

In solchen Fällen wurde bereits vorgeschlagen, eine Art Stent zu setzen, der die Öffnung des Aneurysmas "vergittert" und dadurch die Ausschwemmung der Okklusionsspiralen verhindert. Derartige Stents, die über eine relativ weitmaschige Wandung verfügen, haben aber eine Reihe von Nachteilen.

Zum einen ist dies die weitmaschige Struktur, die den Blutzutritt in das Aneurysma unbeeinträchtigt zulässt. Ist das Aneurysma aber nicht hinreichend mit dem Okklusionsmittel ausgefüllt, bleibt der Druck auf die Gefäßwandung unvermindert bestehen. Eine Nachbehandlung ist unter diesen Umständen nur schwer möglich, da der Stent den Zugang zum Aneurysma beeinträchtigt und die Einbringung weiterer Okklusionsmittel behindert.

Ein weiterer Nachteil ist die fehlende Anpassbarkeit des Stents an seinen Einsatzort. Für eine optimale Funktion sollte sich der Stent dicht an die Gefäßwandung anlegen, ohne jedoch einen übermäßigen Druck auf die Wandung auszuüben. Im Gegensatz zu Stents, die eine Aufweitung des Gefäßes bei Stenosen bewirken sollen, sind diese Stents eher als eine Art Manschette zu verstehen, die das Gefäßlumen und die Endothelwand des Gefäßes möglichst wenig beeinflussen soll. In der Folge sind diese Stents, selbst wenn sie für den Einsatzzweck speziell ausgewählt wurden, nur eingeschränkt an die Anforderungen angepasst.

Aus Drahtgeflecht bestehende Stents sind insbesondere für den Einsatz im koronaren Bereich seit langem bekannt. Diese Stents werden in der Regel als Rundgeflecht gefertigt, wobei die einzelnen Drahtfilamente in gegenläufigen spiralförmigen bzw. helixförmigen Lagen die Stentwandung ausbilden. Es entsteht ein Maschengeflecht, das in radialer Richtung sowohl abstützt als auch für Blut durchlässig ist.

Derartige als Rundgeflecht aus Filamenten bestehende Stents werden beim Einsatz zur Behandlung von Stenosen häufig mit Hilfe eines Ballons am Einsatzort hydraulisch aufgeweitet und an der Gefäßwandung fixiert. Während der Einbringung dient der an einem Einführdraht befestigte Ballon als Transportvehikel, auf das der Stent aufgecrimpt ist. Ein solches Transportvehikel sollte aber für Implantate, die zur Beeinflussung bzw. Kanalisierung des Blutflusses im zerebralen Bereich dienen, nicht eingesetzt werden; vielmehr ist hier ein Implantat von Vorteil, das sich selbständig an den Gefäßdurchmesser anpasst und an die Gefäßwandung anlegt.

In der WO 2008/107172 A1 wird ein Implantat beschrieben, bei dem das Geflecht in einem Mikrokatheter eine gelängte Form mit vermindertem Durchmesser aufweist und am Implantationsort unter Anpassung an den Gefäßdurchmesser und Zunahme der Geflechtdichte expandiert, wobei die an den Implantatenden herausstehenden Filamentenden zumindest paarweise zusammengeführt und miteinander verbunden sind. Auf diese Weise wurde ein Implantat zur Verfügung gestellt, das in der Lage ist, sich an den jeweiligen Gefäßdurchmesser anzupassen, wobei die Filamentenden atraumatisch sind.

Gemäß diesem Stand der Technik sind an den zusammengeführten Filamentenden Verbindungselemente angebracht, die mit Halteelementen nach dem Schlüssel-Schloss-Prinzip zusammenwirken. Das Halteelement, über welches das Implantat mit einem Einführdraht gekoppelt ist, weist Ausnehmungen auf, in die die Verbindungselemente eingepasst sind. Die Verbindungselemente weisen eine Verdickung, beispielsweise eine Kugelform auf, so dass sie in den Ausnehmungen des Halteelements formschlüssig gehalten werden. Die Fixierung in den Ausnehmungen kann mit Hilfe eines Schlauchs erfolgen, der formschlüssig über das Halteelement mit den eingepassten Verbindungselementen gezogen ist. Dieser Schlauch wird nach Erreichen der Endposition des Implantats in Richtung proximal zurückgezogen, und das Implantat wird abgelöst. Anschließend können Einführdraht mit Halteelement, Schlauch und Katheter zurückgezogen und aus dem Körper entfernt werden.

Bei der Einführung eines solchen Implantats in das Blutgefäßsystem ist eine hohe Flexibilität des Gesamtsystems, insbesondere auch des Einführdrahtes und des Schlauchs von Vorteil. Dies gilt insbesondere für den intrakraniellen Bereich mit sehr kleinen Blutgefäßen. Um den Schlauch möglichstflexibel zu machen, kann grundsätzlich ein Schlauch mitgeringerwandstärkeund kleinem Außendurchmesser verwendet werden, hier hat sich jedoch als problematisch herausgestellt, dass sich der Schlauch beim Zurückziehen in Längsrichtung dehnen kann. Die Bewegung des Schlauchs an einem Ende wirdsomitnicht 1:1 an das andere Ende weitergegeben. Infolgedessen werden unter Umständen die Verbindungselemente nicht wie gewünscht vom Halteelement freigegeben, da das distale Endedes Schlauchs die Ausnehmungen des Halteelements trotz der Ausübung einer Kraft am proximalen Ende nachwievor überdeckt.

Ein anderes System zur Freisetzung eines Implantats wird in der US 2006/0259120 A1 beschrieben. Es handeltsich um einen RX(Rapid Exchange)-Katheter, bei dem zur Freisetzung des Implantats eine äußere Umhüllung zurückgezogen wird. Diese weist im Bereich des Implantats bis zum RX-Port einen größeren Außendurchmesser auf als proximal des RX-Ports.

Es stellt sich somit die Aufgabe, ein Implantat der eingangs genannten Art zur Verfügung zu stellen, das einerseits ausreichend flexibel ist, um auch durch englumige Blutgefäße bzw. einen Mikrokatheter mit kleinem Innendurchmesser geführt zu werden, und andererseits eine problemlose Ablösung des Implantats durch Zurückziehen des Schlauchs erlaubt.

Diese Aufgabe wird erfindungsgemäß gelöst durch eine Vorrichtung zur Einbringung eines Implantats für Blutgefäße in den menschlichen Körper, umfassend ein Implantat, einen Einführdraht und eine schlauchartige Umhüllung, wobei das Implantat in der Weise verformbar ist, dass es in einem Mikrokatheter eine Form mit vermindertem Durchmesser annimmtund am Ort der Implantation nach Wegfall des äußeren Zwangs durch den Mikrokatheter unter Anpassung an den Blutgefäßdurchmesserexpandiert, wobei das Implantat am proximalen Ende über Verbindungselemente zu einem Halteelement verfügt, über welches das Implantat an den Einführdraht gekoppelt ist, und das Halteelementperipher Ausnehmungen aufweist, in die die Verbindungselemente eingepasstsind, wobei die schlauchartige Umhüllung formschlüssig über das Halteelementmitden eingepassten Verbindungselementen gezogen ist, so dass die Verbindungselemente in den Ausnehmungen des Halteelements fixiert werden und durch Zurückziehen der schlauchartigen Umhüllung in proximaler Richtung eine Ablösung des Implantats erfolgt, wobei der Außendurchmesser der schlauchartigen Umhüllung zwischen proximalem Ende und distalem Ende variiert und wobei die schlauchartige Um hüllung einen distalen Abschnitt, einen sich in proximaler Richtung anschließenden mittleren Abschnitt mit kleinem Außendurchmesser und einen sich in proximaler Richtung an den mittleren Abschnitt anschließenden proximalen Abschnittmitgroßem Außendurchmesser aufweist.

Durch eine Variation des Außendurchmessers der schlauchartigen Umhüllung zwischen proximalem und distalem Ende werden die Vorteile einer hohen Flexibilität und einer problemlosen, vorhersehbaren Ablösbarkeit miteinander kombiniert. In Teilabschnitten der Umhüllung, insbesondere in einem Bereich, der sich proximal an den distalen Abschnittanschließt, welcher unmittelbar das Halteelement umhüllt, ist eine hohe Flexibilität von besonderer Bedeutung, damitdie Gesamtvorrichtung beim Einführen auch feinen Gefäßwindungen folgt Aus diesem Grund ist hier ein kleiner Außendurchmesser sinnvoll. Auf der anderen Seite sollten die weiter proximal liegenden Abschnitte der schlauchartigen Umhüllung eine ausreichende Widerstandskraft gegen eine unerwünschte Längung aufweisen. Dies ist im proximalen Abschnitt von besonderer Bedeutung, da dieser den größten Teil der Gesamtlänge der Umhüllung ausmacht, die Dehnbarkeit in Längsrichtung sollte daher möglichst gering sein, da sich ansonsten über die Gesamtlänge eine unerwünscht hohe Gesamtdehnung ergeben kann. Auch im distalen Abschnitt selbst, der das Halteelement umschließt, kann eine größere Widerstandskraft gegen eine unerwünschte Längung von Vorteil sein, damit sich dieser Abschnitt der Umhüllung beim Zurückziehen tatsächlich nach proximal bewegtund nichtnurin Längsrichtung dehnt. Aus diesem Grund kann auch der distale Abschnitt einen größeren Außendurchmesser aufweisen als der mittlere Abschnitt, was aber nicht unbedingt erforderlich ist. Der wünschenswerte Außen- und Innendurchmesserim distalen Abschnitt hängtauch mit den Dimensionen des umschlossenen Halteelements zusammen.

Zur Platzierung des Implantats wird zunächst mit Hilfe des Einführdrahtes das Implantat durch den Mikrokatheter bis an die gewünschte Position vorgeschoben. Das proximale Ende des Implantats, an dem sich die Verbindungselemente befinden, die in den Ausnehm ungen des Halteelements gehalten werden, istdabei ebenso wie das Halteelementselbstund zum eist der gesamte Einführdraht von einer schlauchartigen Umhüllung umgeben. Sobald die Freisetzung des Implantats erfolgen soll, wird zunächst der Mikrokatheter zurückgezogen. Dies allein bewirkt jedoch noch keine endgültige Ablösung, da die schlauchartige Umhüllung des Halteelements weiterhin dafür sorgt, dass die Verbindungselemente in den Ausnehmungen des Halteelements gehalten werden. Die Ausnehmungen sind im Außenbereich des Halteelements angeordnet; durch die Aufweitung des Implantats nach Freisetzung aus dem Mikrokatheter besteht somit eine natürliche Tendenz der Verbindungselemente, sich nach außen zu bewegen und sich aus den Ausnehmungen zu lösen. Dies ist jedoch erst dann möglich, wenn auch die schlauchartige Umhüllung zurückgezogen wurde. Der behandelnde Arzt hat somit selbst nach Zurückziehen des Mikrokatheters noch ausreichend Zeit, die Situation zu beurteilen und entweder die endgültige Ablösung des Implantats durch Zurückziehen der Umhüllung in Richtung proximal herbeizuführen oder, falls die Platzierung des Implantats nicht wie gewünscht erfolgt ist, das Implantat durch Zurückziehen des Einführdrahtes erneut in den Mikrokatheter zu bewegen und an anderer Stelle zu implantieren oder auch die Vorrichtung wieder aus dem Körper herauszuholen. Sobald das Implantat an der korrekten Position erfolgreich abgelöst wurde, können der Einführdraht mit dem Halteelement sowie die schlauchartige Umhüllung in den Mikrokatheter zurückgezogen und mit diesem gemeinsam aus dem Blutgefäßsystem entfernt werden.

Im Rahmen der Beschreibung wird unter dem Begriff proximal dem behandelnden Arzt zugewandt verstanden, d. h. das proximale Ende weist in Richtung Körperäußeres. Umgekehrt bedeutet distal dem Arzt abgewandt, das distale Ende liegt also in Richtung Körperinneres.

Typischerweise erstreckt sich die schlauchartige Umhüllung vom Halteelement aus, dessen Ausnehmungen abgedeckt sein müssen, um die Verbindungselemente sicher in den Ausnehmungen zu halten, nach proximal bis außerhalb des Körpers. Denkbar ist aber auch, dass die Umhüllung nicht den gesamten Einführdraht abdeckt, ausreichend ist eine Erstreckung der Umhüllung über das Halteelement. Der Rückzug der Umhüllung erfolgt in diesem Fall über einen zweiten Draht oder Faden, der parallel zum Einführdraht von der Umhüllung aus in Richtung proximal verläuft.

Von Vorteil ist entsprechend eine schlauchartige Umhüllung mit einem distalen Abschnitt, der u. a. das Halteelement umschließt, einem sich in proximaler Richtung anschließenden mittleren Abschnitt mit kleinem Außendurchmesser und einem sich in proximaler Richtung an den mittleren Abschnitt anschließenden proximalen Abschnitt mit großem Außendurchmesser. Darüber hinaus kann es sinnvoll sein, wenn auch der distale Abschnitt einen großen Außendurchmesser aufweist, um das Halteelement mit den eingepassten Verbindungselementen zu umschließen. Mit anderen Worten weist der Abschnitt, der die Ausnehmungen im Halteelement abdeckt, einen großen Außendurchmesser und damit eine größere Steifigkeit auf als der sich in Richtung proximal daran anschließende mittlere Abschnitt, dessen Flexibilität beim Einführen der Vorrichtung von besonderer Bedeutung ist. Der mit Abstand längste Abschnitt, der hier als proximaler Abschnitt bezeichnet wird, weist wiederum einen großen Außendurchmesser auf, um ein Einführen und Zurückziehen der Umhüllung auch über größere Distanzen zu ermöglichen.

Typischerweise liegt die Länge des mittleren Abschnitts bei 50 bis 500 mm, insbesondere 80 bis 120 mm, besonders bevorzugt bei ca. 100 mm. Der distale Abschnitt kann bspw. eine Länge von 2 bis 10 mm haben; dies ist in der Regel ausreichend, um die Ausnehmungen im Halteelement zu überdecken. Die Gesamtlänge der Umhüllung kann 1.000 bis 2.000 mm, z. B. 1.800 mm betragen, entsprechend ist der proximale Abschnitt normalerweise der längste und weist eine Länge von 500 bis 1.900 mm auf.

Die Ausdrücke "großer Außendurchmesser" und "kleiner Außendurchmesser" sind erfindungsgemäß so zu verstehen, dass in Bereichen mit großem Außendurchmesser der Außendurchmesser größer ist als in Bereichen mit kleinem Außendurchmesser. Die genauen Maße können ebenso variieren wie das Verhältnis der Durchmesser, insbesondere in Abhängigkeit von den Verhältnissen im Blutgefäßsystem und dem konkreten Einsatzzweck. Ein typischer großer Außendurchmesser liegt aber in einem Bereich von 0,4 bis 0,8 mm, insbesondere 0,5 bis 0,7 mm, beispielsweise ca. 0,6 mm. Ein typischer kleiner Außendurchmesser liegt bei 0,3 bis 0,55, insbesondere 0,4 bis 0,5, beispielsweise ca. 0,45 mm.

An den proximalen Abschnitt der schlauchartigen Umhüllung mit in der Regel großem Außendurchmesser kann sich noch ein proximales Ende anschließen, das wiederum einen verhältnismäßig kleinen Außendurchmesser aufweist. Hier wird die schlauchartige Umhüllung sinnvollerweise auf den Einführdraht geklemmt, z. B. mit Hilfe eines Torquers, um einen Reibschluss zu erzeugen und eine unerwünschte gegenseitige Verschiebung von Einführdraht und Umhüllung zu verhindern. Ein Verschieben darf bei Verwendung des erfindungsgemäßen Implantats erst dann stattfinden, wenn die Ablösung des Implantats erfolgen soll.

Um das Zurückziehen der schlauchartigen Umhüllung zwecks Freisetzung des Implantats zu vereinfachen, kann am proximalen Ende der Umhüllung, unabhängig vom Außendurchmesser in diesem Bereich, eine Griffmöglichkeit vorgesehen sein. Diese kann in Form einer Verdickung oder als das proximale Ende der Umhüllung umgebende Hülse vorliegen. Wenn die Ablösung des Implantats erfolgen soll, wird der Torquer, der die Umhüllung auf den Einführdraht klemmt, typischerweise gelöst und ggf. am Einführdraht neu angesetzt, um diesen besser handhabbar zu machen. Die Umhüllung kann dann durch den Benutzer an der Griffmöglichkeit gefasst und in Richtung proximal zurückgezogen werden.

Die Passage des Implantats nebst Führungsdraht und umgebender schlauchartiger Umhüllung durch den Katheter kann dadurch erleichtert werden, dass eine Beschichtung der schlauchartigen Umhüllung auf der Außenseite vorgesehen wird, die die Reibung zwischen Umhüllung und Katheter verringert. Hierbei handelt es sich bevorzugt um eine hydrophile Beschichtung.

Beim Zurückziehen der schlauchartigen Umhüllung ist es darüber hinaus sinnvoll, die Reibungskräfte zwischen Einführdraht und Umhüllung möglichst gering zu halten. Hierfür kann, zumindest in Teilbereichen, eine reibungsmindernde Beschichtung auf der Außenseite des Einführdrahtes bzw. der Innenseite der schlauchartigen Umhüllung zum Einsatz kommen. Bevorzugt ist die Verwendung von Polytetrafluorethylen (PTFE). Dies gilt insbesondere für Bereiche, in denen der Einführdraht angeschliffen ist, was typischerweise am proximalen Ende der Fall ist, um das Ergreifen mithilfe eines Torquers zu ermöglichen.

Gemäß einer bevorzugten Ausführungsform variiert nicht nur der Außendurchmesser, sondern auch die Wandstärke der schlauchartigen Umhüllung, d. h. in den Bereichen mit großem Außendurchmesser weist die Umhüllung eine höhere Wandstärke auf als in den Bereichen mit kleinem Außendurchmesser. Die Verringerung der Wandstärke erhöht die Flexibilität und Biegsamkeit der Umhüllung zusätzlich, so dass sie innerhalb des Mikrokatheters feinen Verzweigungen des Blutgefäßsystems besonders gut folgen kann.

Gemäß einer besonders bevorzugten Ausführungsform wird die schlauchartige Umhüllung dadurch hergestellt, dass man von einer einheitlich aufgebauten Umhüllung ausgeht, die zumindest über den größten Teil der Länge einen konstanten Außen- und Innendurchmesser, d. h. auch eine konstante Wandstärke aufweist. Von dieser Umhüllung wird außen in den gewünschten Abschnitten Material abgetragen und somit der Außendurchmesser verringert. Da innen kein Material abgetragen wird, verringert sich in gleichem Maße die Wandstärke der Umhüllung. Man erhält somit eine schlauchartige Umhüllung, die einstückig ist, wobei in Teilabschnitten, insbesondere im mittleren Abschnitt der Außendurchmesser sowie die Wandstärke durch Materialabtrag verringert wurden. In anderen Teilabschnitten, z. B. im proximalen und ggf. im distalen Abschnitt, wird normalerweise kein Material entfernt, d. h. der ursprüngliche Außendurchmesser bleibt hier erhalten.

Das Abtragen von Material kann mit Hilfe von grundsätzlich aus dem Stand der Technik bekannten Verfahren erfolgen, z. B. durch Abdrehen, Abschleifen oder Abschälen mit Hilfe mechanischer Werkzeuge oder auch mit Hilfe eines Lasers. Auch am proximalen Ende kann Material entfernt werden, um hier das Ergreifen durch einen Torquer zu ermöglichen.

Die schlauchartige Umhüllung ist typischerweise aus einem Kunststoff gefertigt. Besonders bewährt haben sich Polyimide. Denkbar ist jedoch auch die Verwendung anderer Materialien, beispielsweise Polypropylen oder Polytetrafluorethylen (PTFE). Auch Kombinationen verschiedener Kunststoffe oder mehrschichtige, koextrudierte Polymere können verwendet werden. Darüber hinaus kann die schlauchartige Umhüllung auch zusätzlich eine Bewehrung aufweisen, indem Fasern, beispielsweise Metallfasern in die Umhüllung eingebettet werden. Denkbar ist z. B. eine schlauchartige Umhüllung, die durch ein Gewebe oder Geflecht verstärkt wird.

Daneben kann die schlauchartige Umhüllung auch aus Metall gefertigt sein, wobei die Umhüllung dünnwandig ausgestaltet sein sollte, um keine zu große Biegesteifigkeit herbeizuführen. Insbesondere bieten sich als Metall Nickel-Titan-Legierungen wie Nitinol an.

Um die Biegesteifigkeit weiter zu verringern kann die schlauchartige Umhüllung Ausnehmungen oder Materialverdünnungen aufweisen, bspw. in Form von Schlitzen oder Öffnungen. Dies gilt unabhängig davon, aus welchem Material die schlauchartige Umhüllung gefertigt ist, d. h. sowohl für Kunststoffe als auch für Metalle. Die Ausnehmungen/Materialverdünnungen können speziell in bestimmten Bereichen der schlauchartigen Umhüllung vorgesehen werden, in denen eine geringe Biegesteifigkeit von besonderer Bedeutung ist, bspw. im distalen Bereich, oder auch über die gesamte Länge der schlauchartigen Umhüllung angeordnet sein. Die Flexibiltät der Umhüllung wird auf diese Weise erhöht, ohne jedoch die Zugfestigkeit der Umhüllung negativ zu beeinflussen.

Das Abtragen von Material kann in der Weise erfolgen, dass die schlauchartige Umhüllung nach der Bearbeitung eine Vielzahl unterschiedlicher Außendurchmesser aufweist. Insbesondere kann der Übergang von Abschnitten mit großem Außendurchmesser zu Abschnitten mit kleinem Außendurchmesser und umgekehrt graduell erfolgen, beispielsweise über mehrere kleine Stufen, die jeweils geringfügig unterschiedliche Außendurchmesser aufweisen. Ebenso ist ein kontinuierlicher Übergang möglich, so dass sich der Außendurchmesser gleichmäßig verringert bzw. vergrößert. In diesem Fall ist der Übergang konisch. Im Längsschnitt betrachtet kann die Wandung der Umhüllung an den Stellen des Übergangs von großem zu kleinem Außendurchmesser eine Fase, einen schrägen oder auch einen runden oder bogenförmigen Verlauf aufweisen.

Alternativ kann die schlauchartige Umhüllung auch mehrstückig aufgebaut sein. In diesem Fall werden Teilabschnitte der Umhüllung mit unterschiedlichen Außendurchmessern miteinander verbunden, in der Regel stoffschlüssig. Zweckmäßig ist eine Verbindung der Teilabschnitte mittels Klebung.

Beim Verbinden der Teilabschnitte mit unterschiedlichen Außendurchmessern sollten sich die Teilabschnitte überlappen, um eine sichere Verbindung, insbesondere eine ausreichende Haftfläche für die Klebung sicherzustellen. Ggf. kann der Innendurchmesser eines Teilabschnitts mit größerem Außendurchmesser erweitert werden, um das partielle Einführen eines Teilabschnitts mit kleinerem Durchmesser zu ermöglichen. Zusätzlich kann dafür gesorgt werden, dass die Übergänge zwischen den Teilabschnitten möglichst gleichmäßig verlaufen und sich der Außendurchmesser jeweils nicht sprunghaft, sondern sukzessive vergrößert bzw. verkleinert. Zu diesem Zweck können die Teilabschnitte angefast werden, möglich ist auch ein Materialabtrag in anderer Weise. Ebenso möglich ist es, eine gewisse zusätzliche Menge einer geeigneten Masse, bspw. des Klebstoffs aufzubringen, um auf diese Weise einen kontinuierlichen Übergang von großem zu kleinem Außendurchmesser zu erhalten.

Die Teilabschnitte können sich auch über längere Distanzen überlappen, beispielsweise kann eine Schicht der schlauchartigen Umhüllung durchgehend über den größten Teil der Länge der schlauchartigen Umhüllung verlaufen. Möglich ist eine Schicht, die am distalen Ende oder geringfügig proximal des distalen Endes der Umhüllung beginnt und bis zum proximalen Ende der Umhüllung durchgehend verläuft und auf diese Weise einen weitgehend einheitlichen Innendurchmesser der Umhüllung gewährleistet. Ein einheitlicher Innendurchmesser ist fertigungstechnisch von Vorteil. In bestimmten Abschnitten, insbesondere im distalen und proximalen Abschnitt, wird auf die durchgehende Schicht der Umhüllung außen eine äußere Schicht der Umhüllung aufgebracht. Die inneren und äußeren Schichten werden miteinander verbunden, insbesondere verklebt. Dort, wo innere und äußere Schicht miteinander verbunden sind, erhält man somit eine Umhüllung mit größerem Außendurchmesser und höherer Gesamtwandstärke, in den Abschnitten, wo keine äußere Schicht vorhanden ist, ist hingegen der Außendurchmesser und die Wandstärke kleiner. Es hat sich überraschend herausgestellt, dass ein mehrschichtiger Aufbau auch die Abschnitte der Umhüllung mit großem Außendurchmesser, insbesondere den proximalen Abschnitt, flexibler macht. Aufgrund der relativ hohen Wandstärke und damit verbunden großen Querschnittsfläche der Außenwand ist jedoch die Zugfestigkeit hoch. Im Vergleich zu einem einschichtigen Aufbau der Wandung der Umhüllung mit insgesamt gleicher Wandstärke ist somit die Flexibilität höher, die Zugfestigkeit jedoch vergleichbar.

Auch bei dieser Ausführungsform können selbstverständlich die Übergänge zwischen Abschnitten mit großem und kleinem Außendurchmesser kontinuierlich oder in Form mehrerer kleiner Stufen ausgebildet werden. Darüber hinaus kann die schlauchartige Umhüllung neben innerer und äußerer Schicht weitere Schichten aufweisen, die Umhüllung kann also grundsätzlich aus beliebig vielen Schichten aufgebaut sein.

Unabhängig von der genauen Ausgestaltung der erfindungsgemäßen Umhüllung ist der Abstand zwischen dem Einführdraht und der Innenwandung der Umhüllung insofern von Bedeutung, als bei zu großem Abstand, d. h. bei im Verhältnis zum Innendurchmesser der Umhüllung zu dünnem Einführdraht während des Vorschubs im Mikrokatheter ein Umbiegen oder Umknicken erfolgen kann, was im Extremfall den weiteren Vorschub unmöglich macht. Umgekehrt ist ein zu kleiner Abstand von Innenwandung der Umhüllung zum Einführdraht insofern problematisch, als bei Relativbewegungen hohe Reibungskräfte auftreten, die beispielsweise das Zurückziehen der Umhüllung zwecks Ablösung des Implantats behindern können.

Es ist von Vorteil, wenn eine innere Schicht der schlauchartigen Umhüllung zumindest weitgehend von distal nach proximal durchgehend verläuft. Hierunter wird verstanden, dass sich die innere Schicht über mindestens 70 %, vorzugsweise mindestens 80 % und besonders bevorzugt mindestens 90 % der Länge erstreckt. Als innere Schicht wird dabei nicht nur eine Schicht verstanden, die zunächst separat vorliegt und erst nachträglich mit einer äußeren Schicht verbunden wird, sondern auch der innere Teil einer einstückig ausgebildeten Umhüllung, wie sie zuvor beschrieben wurde. Dies führt nicht nur zu einem einheitlichen Innendurchmesser der Umhüllung, sondern darüber hinaus auch dazu, dass unerwünschte Längungen der Umhüllung beim proximalen Zurückziehen weitgehend vermieden werden. Auf der einen Seite sind die Abschnitte, in denen Biegsamkeit von besonderer Bedeutung ist, insbesondere der mittlere Abschnitt, besonders dünn und flexibel, so dass die Umhüllung gut durch enge Blutgefäße geführt werden kann. Auf der anderen Seite sind weitere Abschnitte, insbesondere der proximale und ggf. der distale Abschnitt, hinreichend widerstandsfähig gegen eine unerwünschte Verlängerung der Umhüllung, wenn diese nach proximal zurückgezogen wird. Dies gewährleistet eine sichere und problemlose Ablösung des Implantats.

Auch der Einführdraht kann in unterschiedlichen Abschnitten verschiedene Durchmesser aufweisen. Insbesondere kann der Durchmesser distal kleiner sein als im proximalen Abschnitt, da auch für den Einführdraht distal eine niedrige Biegesteifigkeit von Vorteil ist, damit er innerhalb des Mikrokatheters möglichst gut dem Verlauf des Blutgefäßes folgen kann. Auf der anderen Seite kann ein zu kleiner Durchmesser aber auch dazu führen, dass sich der Einführdraht beim Vorschieben verbiegt, wodurch der weitere Vorschub erschwert oder unmöglich gemacht wird. Es ist daher sinnvoll, den Einführdraht im distalen Abschnitt mit einem kleineren Durchmesser zu versehen, da der Einführdraht hier besonders darauf angewiesen ist, dem Verlauf des Blutgefäßes zu folgen, während im proximalen Abschnitt mehr der problemlose Vorschub im Vordergrund steht. Der Durchmesser kann auch über die Länge des Einführdrahtes mehrfach variieren, wobei an den Übergängen der Durchmesser vorzugsweise gleichmäßig zu- oder abnimmt. Die Übergänge sind somit bevorzugt konisch. Die Variation des Durchmesser des Einführdrahtes kann auch unabhängig von der Variation des Außendurchmessers der schlauchartigen Umhüllung erfolgen; insofern betrifft die Erfindung auch eine Vorrichtung gemäß dem Oberbegriff von Anspruch 1, bei der der Durchmesser des Einführdrahtes zwischen proximalem Ende und distalem Ende variiert.

Wenn auch grundsätzlich ein niedriger Durchmesser im distalen Abschnitt des Einführdrahtes von Vorteil ist, können einzelne Bereiche des Einführdrahtes auch im distalen Abschnitt wiederum einen größeren Durchmesser aufweisen. Dies gilt insbesondere für die Spitze des Einführdrahtes. Bei Einteilung des Einführdrahtes in eine proximale und eine distale Hälfte ist es aber sinnvoll, wenn der Durchmesser in der distalen Hälfte im Durchschnitt kleiner ist als in der proximalen Hälfte.

Die Bereiche des Einführdrahtes mit kleinem Durchmesser können mit einem Polymer, z. B. PTFE umhüllt sein. Auf diese Weise wird Spiel zwischen Einführdraht und schlauchartiger Umhüllung vermieden, wodurch eine unerwünschte Verformung des Einführdrahtes beim Vorschub unterbunden wird. Gleichwohl bleibt der Einführdraht in diesem Abschnitt ausreichend flexibel und biegsam, weil das Polymer den Einführdraht praktisch nicht versteift. Das Polymer kann auch in Form einer den Einführdraht ganz oder in Teilbereichen umgebenden spiralförmigen Wendel vorgesehen sein. Die spiralförmige Wendel kann auch aus einem anderen Material, insbesondere aus Metall bestehen.

Von Vorteil ist, wenn sich der Außendurchmesser der schlauchartigen Umhüllung und der Durchmesser des Einführdrahtes in etwa synchron zueinander vergrößern oder verkleinern. Dies ist auch insofern sinnvoll, als in den gleichen Abschnitten von Umhüllung einerseits und Einführdraht andererseits eine hohe Flexibilität wünschenswert ist. Darüber hinaus wird auf diese Weise gewährleistet, dass der Abstand zwischen Innenwandung der Umhüllung und Einführdraht relativ konstant bleibt. Der Durchmesser des Einführdrahtes kann distal durchaus erheblich abnehmen, so dass auch der Innendurchmesser der Umhüllung in den entsprechen Abschnitten klein sein kann; beispielsweise ist es möglich, dass die Umhüllung im mittleren Abschnitt einen kleineren Innendurchmesser aufweist als der Durchmesser des Einführdrahts im proximalen Abschnitt.

Der Einführdraht kann sich nicht nur durch die schlauchartige Umhüllung, sondern darüber hinaus auch durch das eigentliche, zur Ablösung bestimmte Implantat erstrecken. Insbesondere kann sich der Einführdraht nach distal auch über das distale Ende des Implantats hinaus erstrecken, wenn sich das Implantat im komprimierten Zustand befindet, d. h. am Halteelement fixiert ist. Mit anderen Worten liegt die Einführdrahtspitze weiter distal als das distale Ende des Implantats, wenn dieses noch nicht vom Halteelement abgelöst wurde. Auf diese Weise wird bewirkt, dass auch nach Freisetzung des Implantats zunächst noch ein Objekt durch das Innere des Implantats verläuft, solange der Einführdraht nicht zurückgezogen wird. Dies ermöglicht die erneute Sondierung des Gefäßes bzw. Implantates, beispielsweise indem ein Katheter über den Einführdraht und die sich anschließende Einführdrahtspitze geführt wird. Der Katheter wird auf diese Weise durch das freigesetzte und expandierte Implantat bewegt. Die Einführdrahtspitze wird erst durch das endgültige Zurückziehen des Einführdrahtes entfernt.

Die Einführdrahtspitze kann ein rotationssymmetrisches Design haben. Der Querschnitt kann rund, oval, rechteckig oder auf grundsätzlich beliebige andere Art geformt sein. Sinnvoll ist es darüber hinaus, die Einführdrahtspitze visualisierbar zu machen, z. B. indem die Einführdrahtspitze selbst zumindest teilweise aus einem röntgensichtbaren Material gefertigt wird und/oder indem die Einführdrahtspitze an ihrem distalen Ende einen röntgendichten Marker aufweist. Hergestellt werden kann die Einführdrahtspitze aus Edelstahl, Nitinol, Platin, Platin/Iridium oder anderen Metallen.

Die Einführdrahtspitze und der eigentliche Einführdraht können einstückig gefertigt sein, d. h. es handelt es sich letztlich um einen durchgehenden Draht. Möglich ist es aber auch, Einführdrahtspitze und Einführdraht getrennt zu fertigen und erst nachträglich miteinander zu verbinden. Dabei können die vorteilhaften Eigenschaften unterschiedlicher Materialien miteinander kombiniert werden, bspw. kann der eigentliche Einführdraht aus Edelstahl mit guter Vorschiebbarkeit, die Einführdrahtspitze hingegen aus einer Nickel-Titan-Legierung wie Nitinol zur Erhöhung der Flexibilität bestehen.

Der Begriff Einführdraht ist weit zu verstehen und muss nicht in jedem Fall einen klassischen Draht bedeuten. Denkbar sind beispielsweise auch längliche Einführhilfen mit einem inneren Hohlraum. In diesem Fall entspricht der oben diskutierte Durchmesser des Einführdrahtes dem Außendurchmesser. Wichtig ist jedoch, dass sich der Einführdraht nach proximal so weit erstreckt, dass der behandelnde Arzt den Einführdraht ergreifen und bewegen kann.

Das zur Ablösung vorgesehene Implantat selbst weist vorzugsweise eine Wandung aus einzelnen, sich überschneidenden Filamenten auf, die ein röhrenförmiges Geflecht ausbilden. Das röhrenförmige Geflecht ist zumeist ein Rundgeflecht und hat einen vom proximalen oder distalen Ende aus betrachtet kreisförmigen Querschnitt. Grundsätzlich sind jedoch auch Abweichungen von der Kreisform möglich, beispielsweise ein ovaler Querschnitt.

Bei den Filamenten, die die Geflechtstruktur bilden, kann es sich um einzelne Drähte aus Metall handeln, möglich ist aber auch das Vorsehen von Litzen, d. h. mehreren Drähten geringen Durchmessers, die zusammen ein Filament bilden und vorzugsweise miteinander verdrillt sind.

Das Implantat wird im Folgenden anhand eines Flow Diverters beschrieben, der geeignet ist, den Blutfluss in einem Gefäß zu beeinflussen, dergestalt, dass arteriovenöse Fehlbildungen, soweit wie möglich vom Blutfluss abgeschottet werden. Bei den Fehlbildungen handelt es sich zumeist um Aneurysmen. Die erfindungsgemäße Vorrichtung ist hierauf jedoch nicht beschränkt und grundsätzlich auch für andere Implantate geeignet, die dafür vorgesehen sind, in Blutgefäße eingebracht und dort abgelöst zu werden, beispielsweise herkömmliche Stents, die eine Stützfunktion ausüben sollen. Besonders vorteilhaft ist die erfindungsgemäße Vorrichtung im Zusammenhang mit solchen Implantaten, die proximal nicht lediglich über ein einzelnes, sondern über mehrere Enden verfügen, was vor allen Dingen bei Implantaten mit einer Geflechtstruktur aus Filamenten der Fall ist, die in mehreren proximalen Enden zusammengeführt werden. Diese Enden des Implantats sollten gleichzeitig abgelöst werden, was erfindungsgemäß problemlos möglich ist.

Das Implantat kann auch dem Verschluss von Gefäßen dienen, die vom Blutkreislauf abgekoppelt werden sollen, weil sie beispielsweise Tumore versorgen. Das Implantat soll bei optimaler Auswahl von Implantatdurchmesser zu Gefäßdurchmesser in der Lage sein, sich an den jeweiligen Gefäßdurchmesser anzupassen. Im Bereich von Erweiterungen und Aussackungen soll es maximal seinen Nenndurchmesser annehmen, d. h. den Durchmesser, den das Implantat ohne Ausübung eines äußeren Zwangs einnimmt.

Die Platzierung des Implantats sollte auf atraumatische Art und Weise ohne die Hilfe eines Ballons erfolgen. Das Halteelement hält das Implantat über seine Verbindungselemente bis zur endgültigen Freisetzung aus dem Mikrokatheter und bis zum Zurückziehen der schlauchartigen Umhüllung zuverlässig fest und ermöglicht auch das Zurückziehen des Implantates in den Mikrokatheter, solange noch keine vollständige Freisetzung erfolgt ist.

Als Material für das Implantat kommen insbesondere Materialien mit hoher Rückstellkraft bzw. Federwirkung in Frage. Dies sind insbesondere Materialien mit superelastischen oder Formgedächtniseigenschaften, beispielsweise Nitinol. Dabei können für die einzelnen Filamente auch Drähte mit unterschiedlichem Durchmesser verwendet werden. Die Vor- und Nachteile von Drähten mit unterschiedlichem Querschnitt können auf diese Weise kombiniert bzw. kompensiert werden. Der Querschnitt der Drähte ist in den meisten Fällen rund, möglich sind aber auch Drähte mit ovalem oder eckigem Querschnitt oder Kombinationen hieraus.

Wichtig ist in jedem Fall, dass das Implantat einerseits in der Lage ist, eine komprimierte Form zwecks Durchführung durch den Mikrokatheter einzunehmen, sich andererseits aber bei Befreiung vom äußeren Zwang des Mikrokatheters automatisch aufzuweiten und sich am Implantationsort an die Gefäßinnenwandung anzulegen. Möglich ist auch die Fertigung des Implantats aus Verbundmaterialien, beispielsweise aus mit Platin ummantelten Nickel-Titan-Drähten oder mit Nickel-Titan ummantelten Platindrähten. Auf diese Weise werden die Formgedächtniseigenschaften der Nickel-Titan-Legierung (Nitinol) mit der Röntgensichtbarkeit des Platins kombiniert.

Der Durchmesser des Implantats im expandierten Zustand liegt typischerweise zwischen 2,5 und 5,0 mm. Die Länge beträgt beispielsweise 20 bis 40 mm

Der Einführdraht kann aus Edelstahl oder auch aus einem Formgedächtniswerkstoff, insbesondere einer Nickel-Titan-Legierung wie Nitinol gefertigt sein. Bei Einführdrähten, deren Durchmesser variiert, ist es sowohl möglich, den Einführdraht aus einem einzelnen Draht zu schleifen, d. h. in den Bereichen kleineren Durchmessers Material abzutragen. Möglich ist aber auch die Zusammenfügung von mehreren einzelnen Drähten zu einem Einführdraht an den Stellen, an denen sich der Durchmesser des Einführdrahtes ändert. Dabei können unterschiedliche Materialien zum Einsatz kommen. Insbesondere ist es möglich, einen Einführdraht aus Edelstahl mit einer Spitze aus einer Nickel-Titan-Legierung am distalen Ende zu versehen.

Wenn das Implantat als Flow Diverter dient, muss es nicht unbedingt, wie es bei üblichen Stents der Fall ist, eine Stützfunktion ausüben. Das Implantat dient vielmehr in erster Linie zur Kanalisierung des Blutflusses im Bereich der Fehlbildungen im Sinne einer Art innerer Manschette. Es soll beispielsweise auch in einem Aneurysma platzierte Okklusionsmittel daran hindern, in die Gefäßbahn ausgeschwemmt zu werden. Darüber hinaus kann der Ein- und/oder Ausfluss von Blut in ein Aneurysma verhindert werden.

Die Implantate werden typischerweise als Geflecht aus einer Mehrzahl von Filamenten gefertigt, wobei das Geflecht im Prinzip einen Endlosschlauch bildet. Die jeweils benötigte Implantatlänge kann dann aus diesem Endlosschlauch abgelängt werden. Die einzelnen Filamente sind dazu spiral- oder helixförmig aufgewickelt, wobei die einzelnen Filamente als Flechtwerk, d. h. einander kreuzend untereinander und übereinander eingebracht werden. In der Regel sind die einzelnen Filamente dabei in zwei einander in einem konstanten Winkel kreuzenden Richtungen gewickelt, die sich beispielsweise in einem Winkel von 90° schneiden. Bevorzugt sind - im spannungsfreien Normalzustand - Winkel von mehr als 90°, insbesondere von 90 bis 160°, wobei die zu den axialen Enden des Implantats hin offenen Winkel gemeint sind. Eine solche steile Wicklung der Einzelfilamente kann, wenn sie hinreichend dicht ist, zu einem Geflecht mit hoher Oberflächendichte führen, das bei axialer Streckung zu erheblich geringeren Durchmessern auseinandergezogen werden kann. Bei Wegfall der Streckkräfte und hinreichender Rückstellkraft des Filamentmaterials nähert sich das Geflecht dem Nenndurchmesser, d. h. dem ursprünglich spannungsfreien Zustand, wieder an und dehnt sich aus, was zu einer engen Anschmiegung an die Gefäßwand am Implantationsort und zu einer Verdichtung der Maschenstruktur an der Wandung führt. Dies gilt insbesondere auch im Bereich von Gefäßerweiterungen. Zusätzlich kann die Oberflächendichte des Geflechts auch durch die angewandte Flechttechnik variiert werden. Beispielsweise kann das Implantat im mittleren Bereich, in dem typischerweise das Aneurysma abgedeckt wird, dichter geflochten sein als in den Endbereichen, so dass eine weitgehende Abdeckung des Aneurysmahalses gewährleistet ist. Auf der anderen Seite wird durch eine geringere Oberflächendichte in den Endbereichen eine hinreichende Flexibilität gewährleistet. Gefäßabzweigungen (Bifurkationen) können bei den Implantaten beispielsweise durch Bereiche einer geringeren Maschendichte berücksichtigt werden. Die Stärke der Filamente beträgt typischerweise 0,01 bis 0,2 mm, insbesondere 0,02 bis 0,05 mm.

In dem Geflecht können an den Implantatenden herausstehende Filamentenden zumindest paarweise zusammengeführt und miteinander dauerhaft verbunden werden. Dies kann beispielsweise durch Verschweißen erfolgen, aber auch durch mechanisches Verklammern, Verdrillen, Verlöten oder Verkleben. Eine Verbindung der Filamentenden kann auch mittels einer aufgesetzten Hülse erfolgen. Diese Hülse kann mit den Filamentenden eine stoffschlüssige Verbindung eingehen, beispielsweise verschweißt oder auch vercrimpt sein. Eine Alternative besteht darin, dass die Hülse so dimensioniert ist, dass an den Filamentenden befindliche Verdickungen daran gehindert sind, durch die Hülse hindurchzurutschen. Die Hülse ist somit relativ zu den Filamenten in axialer Richtung verschiebbar, sie kann aber nicht vollständig abgezogen werden. Des Weiteren ist es vorteilhaft, wenn die Hülsen in axialer Richtung gegeneinander versetzt sind. Auf diese Weise wird erreicht, dass die Hülsen beim Komprimieren des Implantats nicht unmittelbar übereinander zu liegen kommen, so dass das Implantat insgesamt einen geringeren Durchmesser aufweist.

Von Bedeutung ist das Zusammenführen und Verbinden der Filamentenden insbesondere am proximalen Ende des Implantats; es hat sich herausgestellt, dass am distalen Ende des Implantats auch freie Filamentenden unproblematisch sind. Am proximalen Ende können durch das Zusammenführen der Filamentenden gleichzeitig Verbindungselemente zur Festlegung im Halteelement des Einführdrahtes geschaffen werden. Gleichwohl ist es selbstverständlich möglich, auch am distalen Ende des Implantats die Filamentenden zusammenzuführen und miteinander zu verbinden.

Möglich ist auch eine Zusammenführung der Filamente zu ersten Geflechtenden, die wiederum zu zweiten Geflechtenden verbunden sind, wie in der DE 10 2009 006 180 A1 beschrieben.

Dabei oder zusätzlich können die verbundenen Filamentenden atraumatisch umgeformt werden. Insbesondere können die Filamentenden distal und/oder proximal eine atraumatische Verdickung aufweisen, die z. B. ungefähr kugelförmig ist. Die Verdickung kann durch Laserschweißen, Hartlöten, Verkleben, Aufcrimpen o. ä. aus dem Filamentende ausgeformt oder am Filamentende angebracht werden.

Bei den Verdickungen kann es sich zugleich um die Verbindungselemente handeln, die in die Ausnehmungen des Halteelements eingepasst sind und dort formschlüssig gehalten werden. Die Verbindungselemente sind am proximalen Ende des Implantats angeordnet, um dort über das Halteelement eine Verbindung zum Einführdraht herzustellen.

Die Verbindungselemente können dadurch gebildet werden, dass Verdickungen mit definiertem Durchmesser am proximalen Ende des Implantats angeordnet werden, wobei diese durch Umschmelzen mit Hilfe eines Lasers erzeugbar sind. Die Verdickungen können eine kugelige, ovale, rechteckige, quadratische o. ä. Form aufweisen.

An den proximalen Filamentenden können auch Fortsätze angebracht sein, die sich weiter in Richtung proximal erstrecken und an deren Enden sich die Verbindungselemente befinden. Ein solcher Fortsatz kann bspw. ein Draht sein, der am Verknüpfungspunkt von zwei oder mehr Filamentenden angebracht ist und weiter in axialer Richtung verläuft.

Eine Ausgestaltung der Verbindungselemente in anderer als Kugelform ist ebenfalls möglich, beispielsweise in Form von Ankern, Rechtecken oder anderen Formteilen. Die Verbindungselemente funktionieren nach dem Schlüssel-Schloss-Prinzip, d. h. sie wirken mit einem Halteelement zusammen, das peripher entsprechende Ausnehmungen oder Aufnahmen aufweist. Solange das Halteelement mit angefügtem Implantat in gestreckter und im Durchmesser verkleinerter Form innerhalb eines Mikrokatheters geführt wird, werden beide durch die Katheterwand zwangsweise im Verbund gehalten; nach Austritt des Halteelements aus dem Mikrokatheter und Rückzug der schlauchartigen Umhüllung nach proximal weitet sich das Implantat zu seinem Enddurchmesser auf und befreit sich dadurch aus den Aufnahmen des Halteelements. In der Regel ist das Halteelement rotationssymmetrisch und kann z. B. aus Edelstahl oder Nitinol hergestellt sein.

Grundsätzlich sind allerdings auch Ausführungsformen denkbar, bei denen zusätzliche Verbindungselemente am distalen Ende des Implantats vorhanden sind, die von einem weiteren Halteelement gehalten werden. Ein entsprechend ausgebildetes Objekt mit zwei Halteelementen kann die beiden Halteelemente in einem definierten Abstand an ein und demselben Einführdraht aufweisen, wodurch das Implantat bei vorgegebener Länge auch eine definierte Streckung und Spannung erfährt. Auf diese Art und Weise ist sichergestellt, dass keine Überdehnung stattfindet und die Rückstellkräfte nach der Freisetzung im Gefäß voll wirksam werden können. Alternativ ist aber auch eine Fixierung der Halteelemente an zwei separaten Einführdrähten möglich, die eine Einstellung oder Streckung des Implantats durch den behandelnden Arzt oder über eine entsprechende Feststellvorrichtung ermöglicht. Die Verbindungselemente im weiter proximal angeordneten Halteelement werden erst durch Rückzug der schlauchartigen Umhüllung nach proximal abgelöst, die Verbindungselemente im weiter distal angeordneten Halteelement werden entweder ebenfalls durch Rückzug der Umhüllung oder bereits durch Ausschub aus dem Mikrokatheter abgelöst.

Die Platzierung der erfindungsgemäßen Implantate wird in der Praxis unter Röntgenkontrolle erfolgen. Aus diesem Grund sollte das Implantat und ggf. auch der Einführdraht ein röntgendichtes Markermaterial aufweisen, sofern es nicht aus einem röntgendichten Material selbst gefertigt ist. Solche röntgendichten Materialien sind insbesondere Tantal, Gold, Wolfram und Platinmetalle, etwa Pt-Ir-Legierungen, wobei letztere bevorzugt sind. Diese Marker können beispielsweise als Markerelemente in bekannter Weise an die Filamentenden angeheftet werden, aber auch als Markerfilamente in die Geflechtstruktur des Implantates eingeflochten werden. Die Ummantelung einzelner Filamente mit einer Helix oder Draht aus einem röntgendichten Material wie Platin ist ebenfalls möglich. Die Helix bzw. der Draht kann mit den Filamenten verschweißt, verklebt o. ä. sein. Eine weitere Möglichkeit besteht in der Beschichtung oder Füllung der Filamente mit einem röntgendichten Material.

Ebenfalls möglich sind röntgendichte Markierungen in Form von Hülsen, die die zusammengeführten Filamente umschließen. Diese Hülsen können mit den Filamentenden verschweißt oder auch vercrimpt sein. Die röntgendichten Hülsen können mit den oben erwähnten Hülsen zum Zusammenhalten der Filamentenden identisch sein und somit eine Doppelfunktion erfüllen. Auch die Verbindungselemente können aus einem röntgendichten Material gefertigt sein. Möglich ist darüber hinaus das Versehen eines distalen Abschnitts des Einführdrahtes mit einer Wendel aus röntgendichtem Material, bspw. einer Pt-Wendel. Diese ist vorzugsweise sich proximal anschließend an das Halteelement angeordnet.

Denkbar ist auch die Einbringung röntgensichtbarer Substanzen in die schlauchartige Umhüllung. Hierbei kann es sich um röntgensichtbare Partikel handeln, wie sie üblicherweise in der Röntgentechnik als Kontrastmittel eingesetzt werden. Solche röntgendichten Substanzen sind beispielsweise Schwermetallsalze wie Bariumsulfat oder lodverbindungen. Die Röntgensichtbarkeit der Umhüllung ist bei der Einbringung und Lokalisierung des Implantats hilfreich und kann zusätzlich oder anstelle von Markerelementen Verwendung finden.

Das Geflecht kann im Prinzip auf jede bekannte Art und Weise geflochten werden. Es liegt ein- und/oder mehrflechtig vor. Eine enge Flechtigkeit führt insbesondere bei einem dichten Flechtwerk zu einer hohen Beanspruchung der einzelnen Filamente. Insoweit ist die mehrflechtige Ausführung geeignet, Spannung aus dem Flechtwerk zu nehmen, wobei aber eine zu hohe Flechtigkeit zu einem schlechten Verbund im Geflecht führt. Die Flechtigkeit gibt an, an wie vielen sich mit dem Filament kreuzenden Filamenten ein bestimmtes Filament auf derselben Seite vorbeigeführt wird, bevor es die Seite wechselt, um anschließend an einer entsprechenden Zahl von kreuzenden Filamenten auf der anderen Seite vorbeigeführt zu werden. Bei einer zweiflechtigen Ausführung z. B. wird ein Filament nacheinander oberhalb von zwei das Filament kreuzenden Filamenten, dann nacheinander unterhalb von zwei kreuzenden Filamenten geführt.

Die Filamente können insbesondere auch mehrfach gefacht sein. Die Fachung gibt die Anzahl der zusammengefassten, parallellaufenden Einzelfilamente an. Möglich ist eine Einfach- oder Mehrfachfachung, bei der jeweils ein oder mehrere Einzelfilamente parallel laufen. Da bei der Fertigung des Geflechts die Filamente von Spulen zugeführt werden, bedeutet dies, dass von der entsprechenden Spule ein bzw. mehrere Einzelfilamente gleichzeitig dem Dorn, auf dem das Geflecht gefertigt wird, zugeführt werden. Jedes Einzelfilament kann aus einem einzelnen Draht oder auch aus einer Litze von mehreren zusammengefassten und vorzugsweise miteinander verdrillten Einzeldrähten bestehen.

Die Einzeldrähte können den gleichen Durchmesser sowie auch unterschiedliche Durchmesser aufweisen. Auch können die Drähte aus unterschiedlichen Materialien (Nitinol, Cobalt-Chrom-Legierungen, Platin-Legierungen) bestehen. Drähte aus einem röntgendichten Material beispielsweise sorgen für die Röntgensichtbarkeit des Implantats.

Wie vorstehend beschrieben, kommt es bei der spannungsfreien Anordnung der Einzelfilamente im Geflecht darauf an, die Implantatoberfläche möglichst dicht zu gestalten. Da die Flexibilität des Geflechts erhalten bleiben muss, ist eine 100 %ige Oberflächenabdeckung durch die Filamente allerdings allenfalls annähernd möglich. Je nach Anwendung können sich aber auch geringere Oberflächenabdeckungen ergeben bzw. haben sich auch geringere Oberflächenabdeckungen als ausreichend herausgestellt. Bevorzugt ist eine Oberflächenabdeckung im Bereich von 30 bis 80 %, vorzugsweise von 35 bis 70 %.

Zur Verbesserung der Oberflächenabdeckung kann das Geflecht mit einer Folie ummantelt werden, beispielsweise aus Teflon, Silikon oder einem anderen körperverträglichen Kunststoff. Zur Erhöhung der Flexibilität und Dehnbarkeit kann eine solche Kunststofffolie geschlitzt sein, wobei die Schlitzanordnung gestaffelt ist und die Längsrichtung der Schlitze entlang der Umfangslinie des Implantates verläuft. Eine solche Folie kann beispielsweise durch Eintauchen des Implantates in ein entsprechendes flüssiges Folienmaterial (Dispersion oder Lösung) und anschließende Einfügung der Schlitze, beispielsweise mit einem Laser erzielt werden. Durch Eintauchen kann beispielsweise auch eine ganze oder teilweise Füllung der Maschen erreicht werden.

Alternativ ist es möglich, die einzelnen Filamente des Implantats durch Eintauchen in eine Kunststoffdispersion oder Lösung mit einem solchen Kunststoff zu ummanteln und dadurch den Filamentquerschnitt zu erhöhen. In diesem Fall bleiben offene Maschen, jedoch wird die Maschengröße deutlich vermindert.

Das Implantat kann auf an und für sich bekannte Weise beschichtet sein. Als Beschichtungsmaterialien kommen insbesondere solche in Frage, wie sie für Stents beschrieben sind, etwa mit antiproliferativen, entzündungshemmenden, antithrombogenen, das Einwachsen fördernden und/oder die Anlagerung hindernden, hämokompatiblen Eigenschaften. Bevorzugt ist eine Beschichtung, die das Einwachsen des Implantats und die Neointimabildung fördert. Es kann sinnvoll sein, das Implantat außen derart zu beschichten und innen mit einem Mittel, das die Anhaftung mindert, etwa Heparin oder einem Derivat, ASS oder dazu geeigneten Oligosacchariden und Chitinderivaten. Hier geeignet sind ferner Schichten aus Nano-Partikeln, etwa ultradünnen Schichten aus polymerem SiO₂, die die Anhaftung mindern.

Wie schon vorstehend erwähnt, wird die Kombination aus Einführdraht mit Halteelement, schlauchartiger Umhüllung und Implantat durch einen Mikrokatheter geführt. Dabei ist der Durchmesser des Halteelements sowie der Umhüllung so bemessen, dass beides zusammen ohne Weiteres durch einen üblichen Mikrokatheter geführt werden kann. Entsprechend betrifft die Erfindung auch eine Vorrichtung, die neben dem Implantat, der schlauchartigen Umhüllung und dem Einführdraht auch einen Mikrokatheter umfasst, durch den die weiteren Bestandteile an den Zielort gebracht werden können. Zudem kann die Vorrichtung eine Lagerhülse umfassen, in der das Implantat und ggf. schlauchartige Umhüllung und Einführdraht zur Lagerung aufbewahrt werden können. Bei der Anwendung wird das Implantat mit Hilfe des Einführdrahtes aus der Lagerhülse heraus in den Mikrokatheter hineingeschoben, wobei typischerweise ein konisches Übergangsstück zur Anwendung kommt.

Neben dem erfindungsgemäßen Implantat betrifft die Erfindung auch ein Verfahren zur Herstellung einer schlauchartigen Umhüllung, die in Kombination mit einer Vorrichtung, wie zuvor beschrieben, verwendet werden kann. Die Herstellung kann so erfolgen, dass ausgehend von einer Umhüllung mit einheitlichem Außendurchmesser und einheitlicher Wandstärke in Teilabschnitten der Umhüllung, insbesondere im mittleren Abschnitt, durch Abtragen von Material der Außendurchmesser und die Wandstärke verringert werden. Alternativ ist es auch möglich, die Umhüllung herzustellen, indem mindestens ein Teilabschnitt der Umhüllung mit kleinem Außendurchmesser mit Teilabschnitten der Umhüllung mit großem Außendurchmesser verbunden wird. Die Verbindung erfolgt vorteilhafterweise durch Verkleben.

Die Erfindung wird durch die nachfolgenden Darstellungen näher erläutert. Es zeigt:
- Figur 1a,b: Eine Vorrichtung mit distaler Einführdrahtspitze;
- Figur 2a,b: eine Vorrichtung ohne distale Einführdrahtspitze;
- Figur 3a,b: Varianten zur Verbindung der Filamentenden;
- Figur 4: die Anbindung eines Implantats am Halteelement sowie die Ablösung;
- Figur 5: eine erfindungsgemäße Ausführungsform, bei der der Außendurchmesser der schlauchartigen Umhüllung variiert mit stufigen Übergängen;
- Figur 6: eine weitere erfindungsgemäße Ausführungsform, bei der der Außendurchmesser der schlauchartigen Umhüllung variiert mit konischen Übergängen;
- Figur 7: eine weitere erfindungsgemäße Ausführungsform, bei der der Außendurchmesser der schlauchartigen Umhüllung variiert, wobei die Umhüllung mehrstückig aufgebaut ist;
- Figur 8: eine weitere erfindungsgemäße Ausführungsform, bei der der Außendurchmesser der schlauchartigen Umhüllung nur im proximalen Abschnitt größer ist;
- Figur 9: eine weitere erfindungsgemäße Ausführungsform, bei der der Außendurchmesser sowie die Wandstärke der schlauchartigen Umhüllung variiert; und
- Figur 10: eine weitere erfindungsgemäße Ausführungsform mit konischer schlauchartiger Umhüllung.

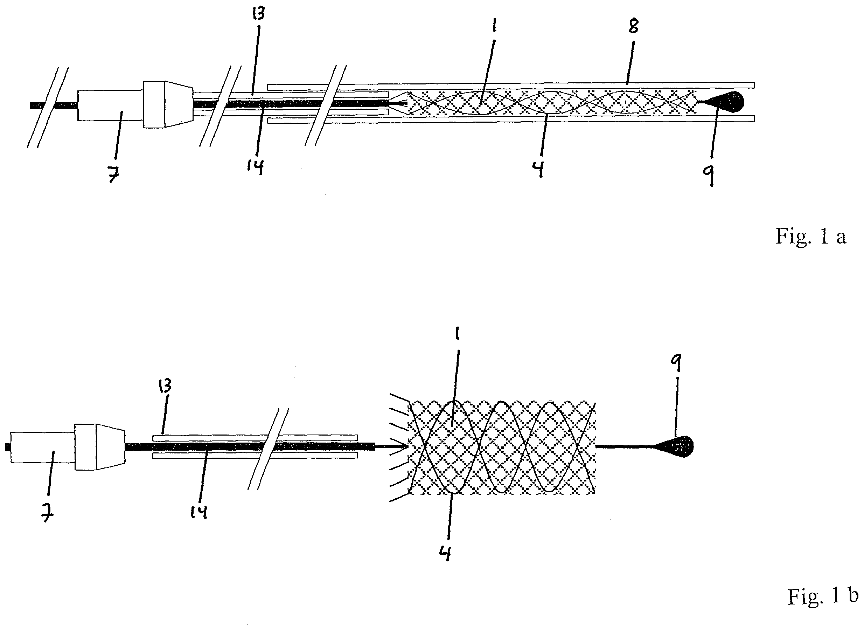

Figur 1a zeigt den grundsätzlichen Aufbau der erfindungsgemäßen Vorrichtung im Lagerungszustand, wobei die Besonderheiten der schlauchartigen Umhüllung 13 in dieser Darstellung nicht zu erkennen sind. Die Vorrichtung setzt sich zusammen aus einem Implantat 1, einem Einführdraht 14 und einer schlauchartigen Umhüllung 13. Das Implantat 1 besteht aus einem Geflecht, in das einzelne Drähte 4 aus einem röntgendichten Material eingeflochten sind, um die Röntgensichtbarkeit des Implantats 1 sicherzustellen. Am proximalen Ende ist das Implantat 1 mit dem Einführdraht 14 gekoppelt, der über ein hier nicht im Detail dargestelltes Halteelement verfügt. Die vom proximalen Ende des Implantats 1 ausgehenden Verbindungselemente sind im Halteelement fixiert, wobei die schlauchartige Umhüllung 13 die Verbindungselemente daran hindert, sich vom Halteelement zu lösen. Der Einführdraht 14 verläuft in Richtung distal durch das Implantat 1 hindurch und weist am distalen Ende eine Einführdrahtspitze 9 auf. Im hier dargestellten Lagerungszustand ist das Implantat 1 von einer Lagerhülse 8 umgeben, aus der das Implantat 1 bei Anwendung in den Mikrokatheter eingeschoben wird. Am proximalen Ende werden Einführdraht 14 und schlauchartige Umhüllung 13 durch einen Torquer 7 zusammengehalten.

In Figur 1b ist das Implantat 1 aus Figur 1a im abgelösten Zustand dargestellt. Die schlauchartige Umhüllung 13 wurde zurückgezogen, so dass die Verbindungselemente sich vom Halteelement des Einführdrahtes 14 lösen konnten. Weiterhin verläuft die Einführdrahtspitze 9 noch durch das Implantat 1 hindurch, kann aber zusammen mit Einführdraht 14 und Umhüllung 13 zurückgezogen werden.

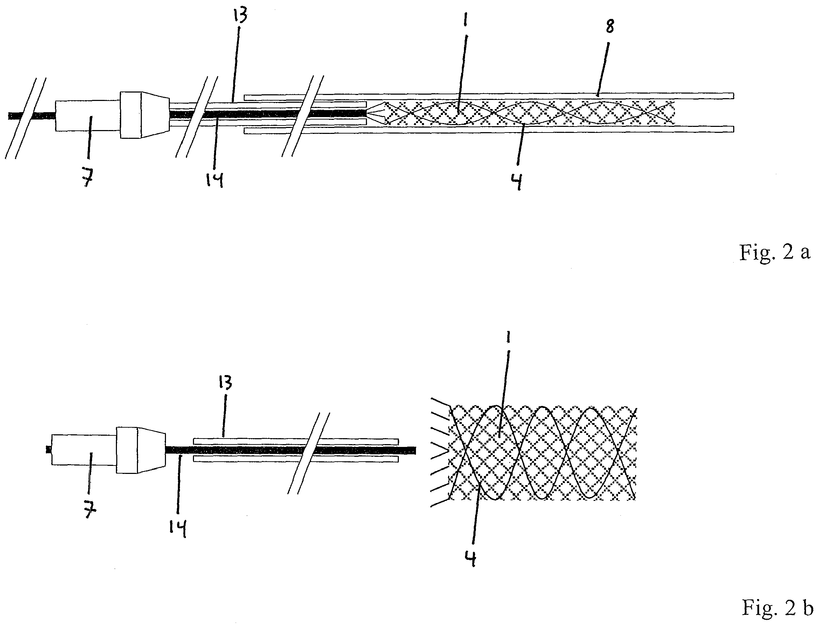

In den Figuren 2a und 2b ist eine Ausführungsform dargestellt, die im Grundsatz der aus den Figuren 1a und 1b entspricht, allerdings wurde hier auf eine distale Einführdrahtspitze 9 verzichtet.

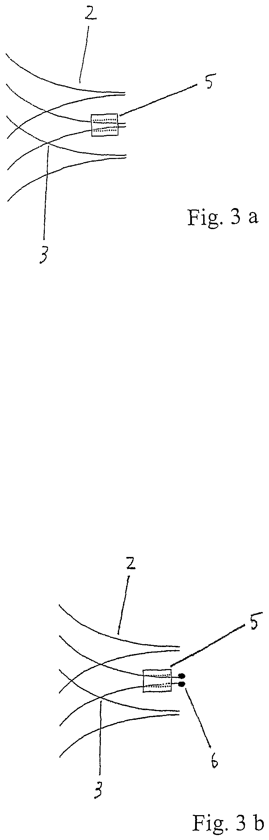

In Figur 3a ist gezeigt, wie die Enden der Filamente 2, die das Geflecht des Implantats 1 ausbilden und sich an den Kreuzungspunkten 3 schneiden, am proximalen Ende durch eine Hülse 5 zusammengehalten werden. Die Hülse 5 kann mit den Filamenten verschweißt oder vercrimpt sein. Darüber hinaus kann die Hülse 5 gleichzeitig der Visualisierung des Implantationsvorgangs dienen, wenn sie aus einem röntgendichten Material gefertigt ist.

Wie in Figur 3b dargestellt, weisen die proximalen Filamentenden atraumatische Verdickungen auf, die als Verbindungselemente 6 dienen. Diese können aus dem Filament 2 selbst gebildet oder zusätzlich angebracht werden. Wenn die Verbindungselemente 6 einen ausreichenden Durchmesser aufweisen, wird die Hülse 5 allein dadurch davon abgehalten, von den Filamentenden abzurutschen. Ebenso möglich ist aber selbstverständlich die Festlegung der Hülse 5 durch Vercrimpen, Verschweißen, Löten, Kleben o. ä.

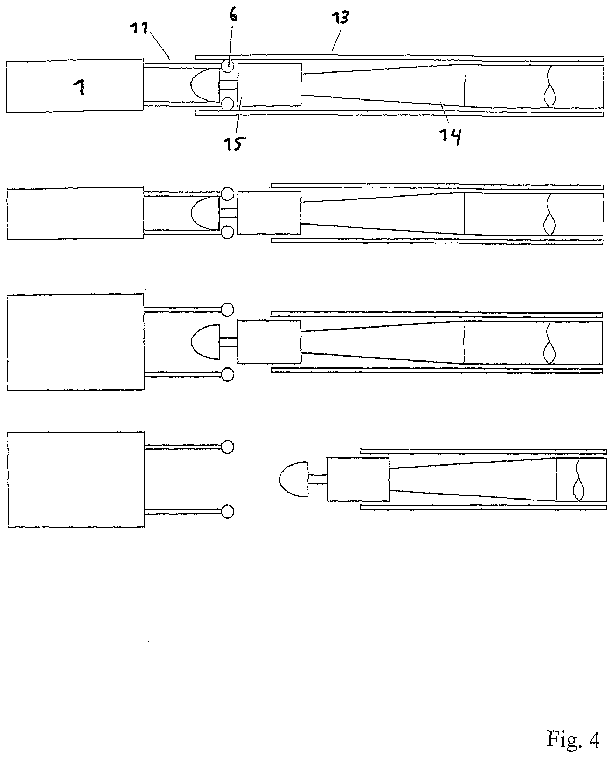

Die Figur 4 zeigt die Festlegung und Ablösung des Implantats 1, das über ein Halteelement 15 mit einem Einführdraht 14 verbunden ist. Halteelement 15 und Einführdraht 14 sind von einer schlauchartigen Umhüllung 13 umgeben. Das Halteelement 15 verfügt über Ausnehmungen, in die die Verbindungselemente 6 am proximalen Ende des Implantats 1 eingreifen. Solange die Umhüllung 13 das Halteelement 15 umgibt, können die Verbindungselemente 6 nicht aus dem Halteelement 15 heraustreten. Sobald jedoch die Umhüllung 13 zurückgezogen wird, kann das Implantat 1 am proximalen Ende expandieren und die Verbindungselemente lösen sich aus den Ausnehmungen im Halteelement 15. Anschließend wird auch der Einführdraht 14, an dessen distalem Ende sich das Halteelement 15 befindet, zurückgezogen.

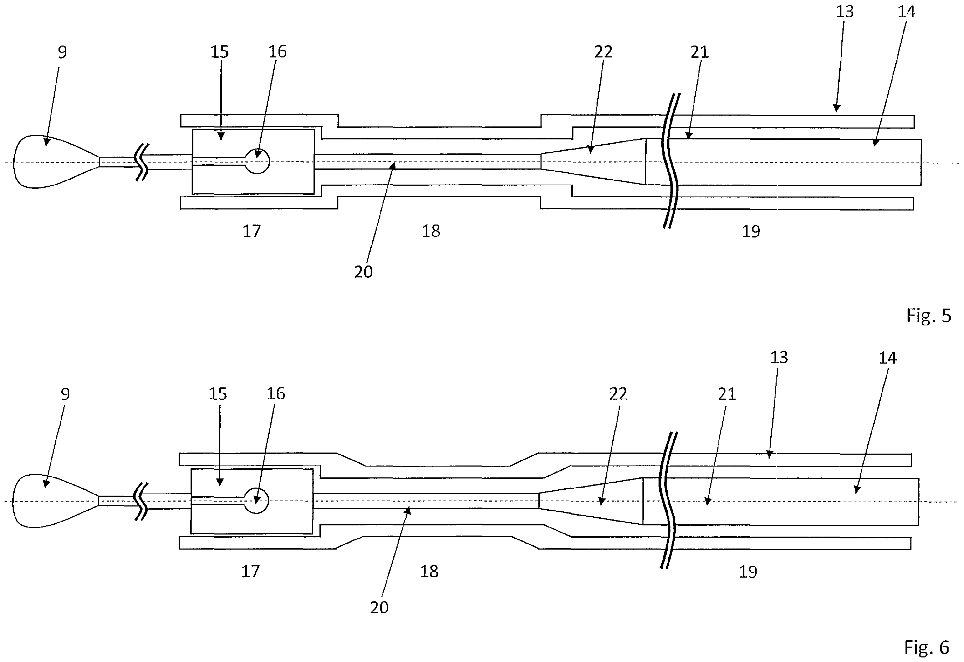

In Figur 5 ist eine erfindungsgemäße Ausführungsform der Vorrichtung dargestellt, wobei das Implantat mit seinen Verbindungselementen der Übersichtlichkeit halber weggelassen wurde. Der Einführdraht 14 verfügt an seinem distalen Ende über eine Einführdrahtspitze 9 sowie über ein Halteelement 15 mit Ausnehmungen 16, die dafür vorgesehen sind, die vom Implantat 1 ausgehenden Verbindungselemente aufzunehmen. Eine schlauchartige Umhüllung 13 umgibt das Halteelement 15 mit den eingepassten Verbindungselementen und hindert so das Implantat an der Ablösung.

Erfindungsgemäß ist von Bedeutung, dass der Außendurchmesser der schlauchartigen Umhüllung 13 variiert. Dabei kann bei der Umhüllung 13 grob zwischen einem distalen Abschnitt 17, der das Halteelement 15 umgibt, einem mittleren Abschnitt 18, der eine hohe Flexibilität und Biegsamkeit aufweisen sollte, und einem deutlich längeren proximalen Abschnitt 19 unterschieden werden. Der mittlere Abschnitt 18 weist einen kleineren Außendurchmesser auf als die beiden anderen Abschnitte 17, 19, wodurch für eine gute Biegsamkeit gesorgt wird.

Zusätzlich variiert auch der Durchmesser des Einführdrahtes 14. Dieser ist im proximalen Abschnitt 21 größer als im distalen Abschnitt 20. Auf diese Weise wird zusätzlich die Flexibilität des Einführdrahtes 14 und damit der Gesamtvorrichtung erhöht, was beim Vorschieben durch den Mikrokatheter in engen Blutgefäßen von Bedeutung ist. Der Übergang 22 zwischen proximalem und distalem Einführdrahtabschnitt ist hier konisch, also fließend, während die Übergänge zwischen den einzelnen Abschnitten der schlauchartigen Umhüllung 13 stufig sind. Diese wird durch plastische Verformung hergestellt.

In Figur 6 ist eine ähnliche Ausführungsform dargestellt, wobei wiederum die schlauchartige Umhüllung 13 durch plastische Verformung erhältlich ist. Im Gegensatz zur Ausführungsform aus Figur 5 sind allerdings die Übergänge zwischen distalem Abschnitt 17 und mittlerem Abschnitt 18 bzw. zwischen mittlerem Abschnitt 18 und proximalem Abschnitt 19 konisch, d. h. die Übergänge erfolgen gleichmäßiger.

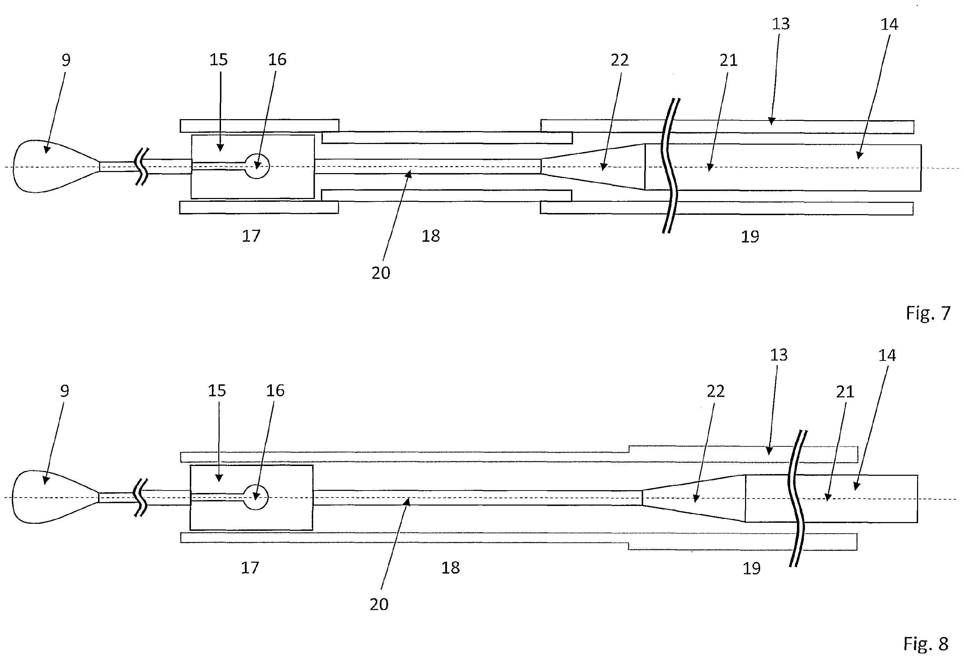

Gemäß Figur 7 ist die schlauchartige Umhüllung 13 mehrstückig aufgebaut und setzt sich aus mehreren Schlauchstücken zusammen, die überlappend verbunden sind. Dabei weist das Schlauchstück, das den mittleren Abschnitt 18 der Umhüllung 13 ausbildet, einen geringeren Durchmesser auf als die Schlauchstücke des distalen und des proximalen Abschnitts 17, 19. Die Verbindung der Schlauchstücke kann insbesondere durch Verkleben erfolgen.

In Figur 8 ist eine Ausführungsform dargestellt, bei der die schlauchartige Umhüllung 13 einstückig ist. Ausgehend von einer Umhüllung mit einheitlichem Außendurchmesser und einheitlicher Wandstärke wird im distalen Abschnitt 17 und im mittleren Abschnitt 18 außen Material abgetragen, so dass Außendurchmesser und Wandstärke in diesen Abschnitten verringert werden. Auf diese Weise wird eine schlauchartige Umhüllung 13 mit hoher distaler Flexibilität und Biegsamkeit erhalten.

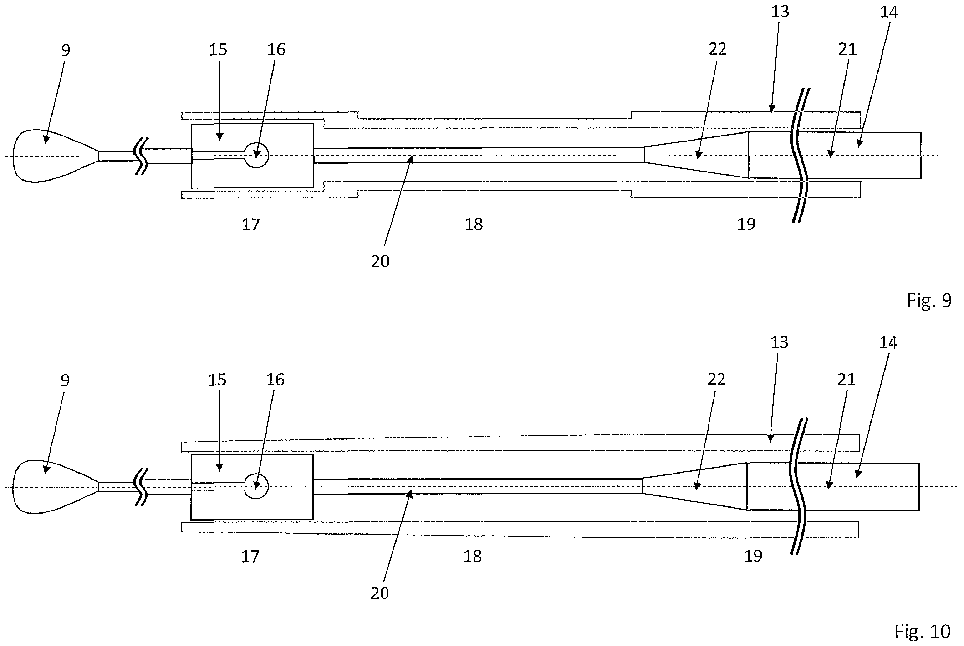

Figur 9 zeigt ebenfalls eine einstückige schlauchartige Umhüllung 13. Im Gegensatz zu Figur 8 weist allerdings der distale Abschnitt 17 einen größeren Außendurchmesser auf als der mittlere Abschnitt 18. Dies kann insbesondere dann sinnvoll sein, wenn das Halteelement 15 einen größeren Durchmesser hat.

In den Figuren 8 und 9 sind die Übergänge zwischen den einzelnen Abschnitten 17, 18 und 19 stufig dargestellt, selbstverständlich sind jedoch auch abgerundete oder angefaste Übergänge möglich. Ebenso ist es möglich, mehrere Stufen an den Übergängen vorzusehen.

In Figur 10 schließlich ist eine Ausführungsform gezeigt, bei der die schlauchartige Umhüllung 13 ebenfalls einstückig ist, bei der jedoch der Außendurchmesser vom proximalen Abschnitt 19 zum distalen Abschnitt 17 kontinuierlich abnimmt, die Umhüllung 13 also eine leichte Konusform aufweist. Im distalen und mittleren Abschnitt 17, 18 wird Material auf der Außenseite der Umhüllung 13 mittels Abdrehen oder Abschleifen abgetragen. Auch hierdurch wird die Biegsamkeit der Umhüllung 13 nach distal hin erhöht.

## Patentansprüche

1. Vorrichtung zur Einbringung eines Implantats (1) für Blutgefäße in den menschlichen Körper, umfassend ein Implantat (1), einen Einführdraht (14) und eine schlauchartige Umhüllung (13), wobei das Implantat(1) in der Weise verformbar ist, dass es in einem Mikrokatheter eine Form mit vermindertem Durchmesserannimmt und am Ort der Implantation nach Wegfall des äußeren Zwangs durch den Mikrokatheter unter Anpassung an den Blutgefäßdurchmesser expandiert, das Implantat (1) am proximalen Ende über Verbindungselemente (6) zu einem Halteelement (15) verfügt, über welches das Implantat (1) an den Einführdraht(14) gekoppelt ist, und das Halteelement (15) peripher Ausnehmungen (16) aufweist, in die die Verbindungselemente (6) eingepasstsind, wobei die schlauchartige Umhüllung (13) formschlüssig über das Halteelement (15) mit den eingepassten Verbindungselementen (6) gezogen ist, sodass die Verbindungselemente (6) in den Ausnehmungen (16) des Halteelements (15) fixiert werden und durch Zurückziehen der schlauchartigen Umhüllung (13) in proximaler Richtung eine Ablösung des Implantats (1) erfolgt, wobei der Außendurchmesser der schlauchartigen Umhüllung (13) zwischen proximalem Ende und distalem Ende variiert, **gekennzeichnet durch** einen distalen Abschnitt (17) derschlauchartigen Umhüllung (13), einem sich in proximaler Richtung anschließenden mittleren Abschnitt (18) mit kleinem Außendurchmesser und einem sich in proximaler Richtung an den mittleren Abschnitt (18) anschließenden proximalen Abschnitt (19) mit großem Außendurchmesser.

2. Vorrichtung nach Anspruch 1, **dadurch gekennzeichnet, dass** die schlauchartige Umhüllung (13) im distalen Abschnitt (17) einen größeren Außendurchmesser aufweist als im mittleren Abschnitt (18).

3. Vorrichtung nach Anspruch 1 oder 2, **dadurch gekennzeichnet, dass** sich an den proximalen Abschnitt (19) der schlauchartigen Umhüllung in proximaler Richtung ein proximales Ende mit kleinem Außendurchmesser anschließt.

4. Vorrichtung nach einem der Ansprüche 1 bis 3, **dadurch gekennzeichnet, dass** in den Abschnitten der schlauchartigen Umhüllung (13) mit großem Außendurchmesser die Wandstärke der Umhüllung (13) höher ist als in den Abschnitten mit kleinem Außendurchmesser.

5. Vorrichtung nach einem der Ansprüche 1 bis 4, **dadurch gekennzeichnet, dass** die schlauchartige Umhüllung (13) einstückig ausgebildet und dadurch erhältlich ist, dass ausgehend von einer Umhüllung (13) mit einheitlichem Außendurchmesser und konstanter Wandstärke in Teilabschnitten, insbesondere im mittleren Abschnitt (18), durch Abtragen von Material der Außendurchmesser und die Wandstärke verringert werden.

6. Vorrichtung nach einem der Ansprüche 1 bis 4, **dadurch gekennzeichnet, dass** die schlauchartige Umhüllung (13) mehrstückig aufgebaut und dadurch erhältlich ist,dass mindestens ein TeilabschnittderUmhüllung (13) mit kleinem Außendurchmesser mit Teilabschnitten der Umhüllung (13) mit großem Außendurchmesser verbunden wird.

7. Vorrichtung nach Anspruch 6, **dadurch gekennzeichnet, dass** die Teilabschnitte der Umhüllung (13) durch Verkleben verbunden werden.

8. Vorrichtung nach Anspruch 6 oder 7, **dadurch gekennzeichnet, dass** sich eine Schicht der Umhüllung (13) mit geringer Wandstärke bis zum proximalen Ende der Umhüllung (13) erstreckt und im proximalen und/oder im distalen Abschnitt (17, 19) von einer äußeren Schicht der Umhüllung (13) umgeben und mit dieser verbunden ist.

9. Vorrichtung nach einem der Ansprüche 1 bis 8, **dadurch gekennzeichnet, dass** der Durchmesser des Einführdrahtes (14) über seine Länge variiert.

10. Vorrichtung nach Anspruch 9, **dadurch gekennzeichnet, dass** der durchschnittliche Durchmesser des Einführdrahtes (14) in der distalen Hälfte geringer ist als in der proximalen Hälfte.

11. Vorrichtung nach einem der Ansprüche 1 bis 10, **dadurch gekennzeichnet, dass** sich das distale Ende des Einführdrahtes (14) weiter nach distal erstrecktals das distale Endedes Implantats (1) im am Halteelement (15) fixierten Zustand.

12. Vorrichtung nach einem der Ansprüche 1 bis 11, **dadurch gekennzeichnet, dass** das Implantat (1) eine Wandung aus einzelnen, sich überschneidenden Filamenten (2) aufweist, die ein röhrenförmiges Geflecht ausbilden.

13. Verfahren zur Herstellung einer schlauchartigen Umhüllung (13), wobei die schlauchartige Umhüllung (13) Teil einer Vorrichtung nach einem der Ansprüche 1 bis 12 ist, **dadurch gekennzeichnet, dass** ausgehend von einer Umhüllung (13) mit einheitlichem Außendurchmesser und konstanter Wandstärke in Teilabschnitten, insbesondere im mittleren Abschnitt (18), durch Abtragen von Material der Außendurchmesser und die Wandstärke verringert werden.

14. Verfahren zur Herstellung einer schlauchartigen Umhüllung (13), wobei die schlauchartige Umhüllung (13) Teil einer Vorrichtung nach einem der Ansprüche 1 bis 12 ist, **dadurch gekennzeichnet, dass** mindestens ein Teilabschnitt der Umhüllung (13) mit kleinem Außendurchmesser mit Teilabschnitten der Um hüllung (13) mitgroßem Außendurchmesser verbunden, insbesondere verklebtwird.

## Claims

1. Device for the introduction of an implant (1) into blood vessels of the human body, said device comprising an implant (1), a pusher or insertion wire (14), and a tube-like sheathing (13), wherein the implant (1) is capable of being deformed inside a microcatheter in a manner that allows it to assume a shape of reduced diameter and, after omission of such external constraint exerted by the microcatheter, expand at the placement site and adapt to the blood vessel diameter, and wherein the implant (1) being provided at the proximal end with connection elements (6) attaching it to a retaining element (15) by means of which the implant (1) is coupled to the pusher wire (14), and wherein the retaining element (15) is provided with peripheral cutouts (16) into which the connecting elements (6) are fitted, with the tube-like sheathing (13) being drawn in a form-closed manner over the retaining element (15) with fitted connection elements (6) such that the connection elements (6) are secured within the cutouts (16) of the retaining element (15) and the implant (1) being released by the retraction of the tube-like sheathing (13) in proximal direction, wherein the outer diameter of the tube-like sheathing (13) varies between the proximal end and the distal end,
**characterized by** a distal section (17) of the tube-like sheathing (13), an adjacently arranged middle section (18) of small outer diameter extending in proximal direction, and a proximal section (19) of large outer diameter arranged adjacent to the middle section (18) and extending in proximal direction.

2. Device according to claim 1, **characterized in that** the outer diameter of the tube-like sheathing (13) is larger in the distal section (17) than in the middle section (18).

3. Device according to claim 1 or 2, **characterized in that** a proximal end of small outer diameter adjoins the proximal section (19) of the tube-like sheathing in proximal direction.

4. Device according to any of claims 1 to 3, **characterized in that** in the sections of the tube-like sheathing (13) of large outer diameter the wall thickness of sheathing (13) is greater than in the sections of small outer diameter.

5. Device according to any of claims 1 to 4, **characterized in that** the tube-like sheathing (13) is of one-piece design and obtained in such a way that based on a sheathing (13) of uniform outer diameter and constant wall thickness said outer diameter and the wall thickness are reduced by the removal of material in partial sections, in particular in the middle section (18).

6. Device according to any of claims 1 to 4, **characterized in that** the tube-like sheathing (13) comprises a plurality of parts and is obtained in such a manner that at least one partial section of the sheathing (13) of small outer diameter is connected to partial sections of the sheathing (13) of large outer diameter.

7. Device according to claim 6, **characterized in that** the partial sections of sheathing (13) are connected by adhesive bonding.

8. Device according to claim 6 or 7, **characterized in that** a layer of the sheathing (13) of small wall thickness extends up to the proximal end of sheathing (13) and, in the proximal and/or distal section (17, 19), is enwrapped by and attached to an outer layer of the sheathing (13).

9. Device according to any of claims 1 to 8, **characterized in that** the diameter of the pusher wire (14) varies over its length.

10. Device according to claim 9, **characterized in that** the average diameter of the pusher wire (14) is smaller in the distal half than in the proximal half.

11. Device according to any of claims 1 to 10, **characterized in that** the distal end of the pusher wire (14) extends further distally than the distal end of the implant (1) in a state fixed to the retaining element (15).

12. Device according any of claims 1 to 11, **characterized in that** the implant (1) has a wall composed of individual filaments (2) intersecting with one another and forming a tubular braiding.

13. Method for the manufacture of a tube-like sheathing (13), wherein the tube-like sheathing (13) is part of a device according to any of claims 1 to 12, **characterized in that** based on a sheathing (13) of uniform outer diameter and constant wall thickness said outer diameter and the wall thickness are reduced by the removal of material in partial sections, in particular in the middle section (18).

14. Method for the manufacture of a tube-like sheathing (13), wherein the tube-like sheathing (13) is part of a device according to any of claims 1 to 12, **characterized in that** at least one partial section of the sheathing (13) of small outer diameter is connected to partial sections of the sheathing (13) of large outer diameter, in particular by adhesive bonding.

## Revendications

1. Dispositif pour l'insertion d'un implant (1) pour des vaisseaux sanguins dans le corps humain, comprenant un implant (1), un fil d'introduction (14) et une gaine du type tuyau flexible (13), dans lequel l'implant (1) est déformable, de telle manière qu'il prenne dans un microcathéter une forme de diamètre réduit et qu'il se dilate à l'endroit de l'implantation, après la disparition de la contrainte extérieure produite par le microcathéter, avec adaptation au diamètre du vaisseau sanguin, l'implant (1) dispose à l'extrémité proximale d'éléments de liaison (6) à un élément de maintien (15) par lequel l'implant (1) est couplé au fil d'introduction (14), et l'élément de maintien (15) présente en périphérie des évidements (16) dans lesquels les éléments de liaison (6) sont insérés, dans lequel la gaine du type tuyau flexible (13) est tirée avec adaptation de la forme sur l'élément de maintien (15) avec les éléments de liaison insérés (6), de telle manière que les éléments de liaison (6) soient fixés dans les évidements (16) de l'élément de maintien (15) et qu'une libération de l'implant (1) se produise par retrait de la gaine du type tuyau flexible (13) en direction proximale, dans lequel le diamètre extérieur de la gaine du type tuyau flexible (13) varie entre l'extrémité proximale et l'extrémité distale, **caractérisé par** une section distale (17) de la gaine du type tuyau flexible (13), une section médiane (18) de petit diamètre extérieur s'y raccordant en direction proximale et une section proximale (19) de grand diamètre extérieur se raccordant en direction proximale à la section médiane (18).

2. Dispositif selon la revendication 1, **caractérisé en ce que** la gaine du type tuyau flexible (13) présente dans la section distale (17) un plus grand diamètre extérieur que dans la section médiane (18).

3. Dispositif selon la revendication 1 ou 2, **caractérisé en ce qu'**une extrémité proximale de petit diamètre extérieur se raccorde en direction proximale à la section proximale (19) de la gaine du type tuyau flexible.

4. Dispositif selon l'une quelconque des revendications 1 à 3, **caractérisé en ce que** l'épaisseur de paroi de la gaine (13) est plus élevée dans les sections de la gaine du type tuyau flexible (13) de grand diamètre extérieur que dans les sections de petit diamètre extérieur.

5. Dispositif selon l'une quelconque des revendications 1 à 4, **caractérisé en ce que** la gaine du type tuyau flexible (13) est réalisée en une seule pièce et il est ainsi réalisable que, à partir d'une gaine (13) de diamètre extérieur unique et d'épaisseur de paroi constante, le diamètre extérieur et l'épaisseur de paroi puissent être réduits dans des sections partielles, en particulier dans la section médiane (18), par enlèvement de matière.

6. Dispositif selon l'une quelconque des revendications 1 à 4, **caractérisé en ce que** la gaine du type tuyau flexible (13) est composée de plusieurs parties et il est ainsi réalisable qu'au moins une section partielle de la gaine (13) de petit diamètre extérieur soit assemblée à des sections partielles de la gaine (13) de grand diamètre extérieur.

7. Dispositif selon la revendication 6, **caractérisé en ce que** les sections partielles de la gaine (13) sont assemblées par collage.

8. Dispositif selon la revendication 6 ou 7, **caractérisé en ce qu'**une couche de la gaine (13) de faible épaisseur de paroi s'étend jusqu'à l'extrémité proximale de la gaine (13) et est entourée dans la section proximale et/ou distale (17, 19) par une couche extérieure de la gaine (13) et est assemblée à celle-ci.

9. Dispositif selon l'une quelconque des revendications 1 à 8, **caractérisé en ce que** le diamètre du fil d'introduction (14) varie sur sa longueur.

10. Dispositif selon la revendication 9, **caractérisé en ce que** le diamètre moyen du fil d'introduction (14) est plus petit dans la moitié distale que dans la moitié proximale.

11. Dispositif selon l'une quelconque des revendications 1 à 10, **caractérisé en ce que** l'extrémité distale du fil d'introduction (14) s'étend plus loin en direction distale que l'extrémité distale de l'implant (1) dans l'état fixé à l'élément de maintien (15).

12. Dispositif selon l'une quelconque des revendications 1 à 11, **caractérisé en ce que** l'implant (1) présente une paroi en filaments individuels qui se coupent (2), qui forment un tissage tubulaire.

13. Procédé de fabrication d'une gaine du type tuyau flexible (13), dans lequel la gaine du type tuyau flexible (13) fait partie d'un dispositif selon l'une quelconque des revendications 1 à 12, **caractérisé en ce que**, partant d'une gaine (13) présentant un diamètre extérieur unique et une épaisseur de paroi constante, on réduit le diamètre extérieur et l'épaisseur de paroi dans des sections partielles, en particulier dans la section médiane (18), par enlèvement de matière.

14. Procédé de fabrication d'une gaine du type tuyau flexible (13), dans lequel la gaine du type tuyau flexible (13) fait partie d'un dispositif selon l'une quelconque des revendications 1 à 12, **caractérisé en ce que** l'on assemble, en particulier on colle, au moins une section partielle de la gaine (13) de petit diamètre extérieur à des sections partielles de la gaine (13) de grand diamètre extérieur.
